(19) Europäisches Patentamt
European Patent Office
Office européen des brevets

(11) Veröffentlichungsnummer : **0 112 293**
**B1**

(12) # EUROPÄISCHE PATENTSCHRIFT

(45) Veröffentlichungstag der Patentschrift : **30.09.87**

(21) Anmeldenummer : **83810578.1**

(22) Anmeldetag : **08.12.83**

(51) Int. Cl.⁴ : **C 07 D213/64**, A 01 N 53/00

(54) **Isomere des 3-(2,2-Dichlorvinyl)-2,2-dimethylcyclopropankarbonsäure-alpha-methyl-(6-phenoxy-2-picolyl)esters, Verfahren zu ihrer Herstellung und ihre Verwendung in der Schädlingsbekämpfung.**

(30) Priorität : **14.12.82 CH 7271/82**
**16.11.83 CH 6157/83**

(43) Veröffentlichungstag der Anmeldung : **27.06.84 Patentblatt 84/26**

(45) Bekanntmachung des Hinweises auf die Patenterteilung : **30.09.87 Patentblatt 87/40**

(84) Benannte Vertragsstaaten : **AT BE CH DE FR GB IT LI NL**

(56) Entgegenhaltungen :
**GB-A- 2 053 903**
**US-A- 4 323 574**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Ackermann, Peter, Dr.**
**Hangelimattweg 113**
**CH-4148 Pfeffingen (CH)**
Erfinder : **Gsell, Laurenz, Dr.**
**Maiengasse 56**
**CH-4056 Basel (CH)**
Erfinder : **Kohler, Boris, Dr.**
**Holzmattstrasse 45**
**CH-4102 Binningen (CH)**

**EP 0 112 293 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue diastereomere 1 R-cis-3-(2,2-Dichlorvinyl)-2,2-dimethylcyclopropankarbonsäure-R,S-α-methyl-(6-phenoxy-2-picolyl) ester der Formel I

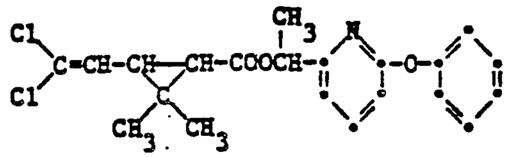

(I)

darunter den 1 R-cis-3-(2,2-Dichlorvinyl)-2,2-dimethylcyclopropankarbonsäure-R-α-methyl-(6-phenoxy-2-picolyl) ester und den 1 R-cis-3-(2,2-Dichlorvinyl)-2,2-dimethylcyclopropankarbonsäure-S-α-methyl-(6-phenoxy-2-picolyl) ester, sowie neue diastereomere 1 R-trans-3-(2,2-Dichlorvinyl)-2,2-dimethylcyclopropankarbonsäure-R,S-α-methyl-(6-phenoxy-2-picolyl) ester der Formel I, darunter den 1 R-trans-3-(2,2-Dichlorvinyl)-2,2-dimethylcyclopropankarbonsäure-R-α-methyl-(6-phenoxy-2-picolyl) ester und den 1 R-trans-3-(2,2-Dichlorvinyl)-2,2-dimethylcyclopropankarbonsäure-S-α-methyl-(6-phenoxy-2-picolyl) ester, Verfahren zu ihrer Herstellung und ihre Verwendung in der Schädlingsbekämpfung.

Die Verbindungen der Formel I werden nach an sich bekannten Methoden z. B. wie folgt hergestellt :

1)

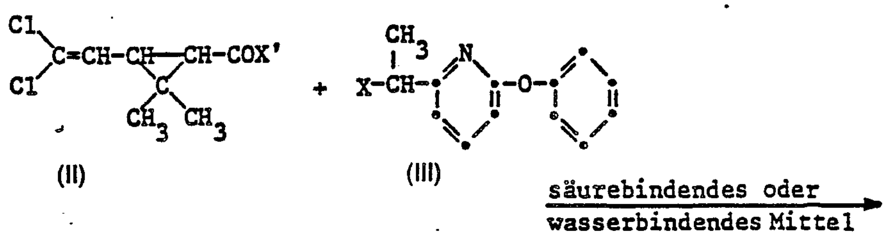


(II) + (III) → (I)

2)

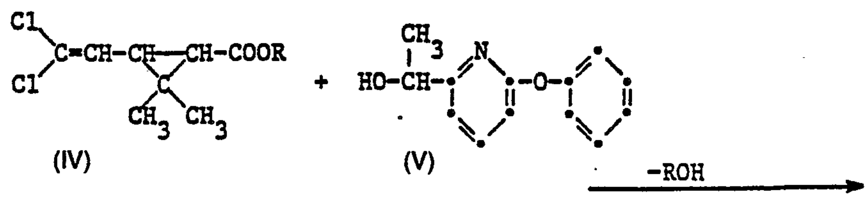


(IV) + (V) → (I)

In den Formeln II und III stehen die Symbole X und X' je für eine Hydroxylgruppe oder eines für eine Hydroxylgruppe und das andere für ein Halogenatom, insbesondere für Chlor oder Brom. In der Formel IV steht R für $C_1$-$C_4$-Alkyl, insbesondere für Methyl oder Aethyl.

Als säurebindende Mittel kommen insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z. B. Kalium-t. butylat und Natriummethylat in Betracht. Als wasserbindendes Mittel kann z. B. Bicyclohexylcarbodiimid verwendet werden. Die Verfahren 1 und 2 werden bei einer Reaktionstemperatur zwischen —10 und 120 °C, meist zwischen 20 und 80 °C, bei normalen oder erhöhtem Druck und vorzugsweise in einem inerten Lösungs- oder Verdünnungsmittel durchgeführt. Als Lösungs- oder Verdünnungsmittel eignen sich z. B. Aether und ätherartige Verbindungen, wie Diäthyläther, Dipropyläther, Dioxan, Dimethoxyäthan und Tetrahydrofuran ; Amide, wie N,N-dialkylierte Carbonsäureamide ; aliphatische, aromatische sowie halogenierte Kohlenwasserstoffe, insbesondere Benzol, Toluol, Xylole, Chloroform und Chlorbenzol ; Nitrile wie Acetonitril ; Dimethylsulfoxid und Ketone wie Aceton und Methyläthylketon.

Die durch die Verfahren 1 und 2 hergestellten Diastereomerengemische können chromatographisch in die optisch reinen Isomeren aufgetrennt werden.

Die Verbindungen II und IV sind als reine Isomere und als Isomerengemische und die Verbindungen der Formeln III und V nur als Isomerengemische bekannt und können analog bekannten Methoden hergestellt werden.

Die Isomere der Formel I eignen sich zur Bekämpfung von verschiedenartigen Schädlingen an Tieren und Pflanzen. Diese Verbindungen können z. B. zur Bekämpfung von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Vor allem eignen sich Verbindungen der Formel I zur Bekämpfung von pflanzenschädigenden Insekten, insbesondere pflanzenschädigenden Frassinsekten, in Zier- und Nutzpflanzen, wie z. B. in Baumwoll- und Reiskulturen (z. B. Spodoptera littoralis, Heliothis virescens, Chilo suppressalis und Laodelphax) sowie Gemüsekulturen (z. B. Leptinotarsa decemlineata).

Die Wirkstoffe der Formel I zeigen auch eine sehr günstige Wirkung gegen Fliegen, wie z. B. Musca domestica und Mückenlarven und gegen ektoparasitäre Milben und Zecken, z. B. der Familien Ixodidae, Argasidae und Dermanyssidae. Daneben zeichnen sich die Verbindungen der Formel I durch eine breite ovizide und ovolarvizide Wirkung aus.

Ueberraschenderweise haben Isomere der Formel I ein insektizides Wirkungsspektrum, welches durch bedeutend geringere Aufwandmengen erzielt wird, als durch die aus dem U.S.P. 4 323 574 bekannten Isomerengemische des 3-(2,2-Dichlorvinyl)-2,2-dimethylcyclopropankarbonsäure-α-methyl-(6-phenoxy-2-picolyl) esters.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z. B. org. Phosphorverbindungen ; Nitrophenole und deren Derivate ; Formamidine ; Harnstoffe ; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u. a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithionate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimmstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8-22 C-Atomen.

Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxy-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben :

« Mc Cutcheon's Detergents and Emulsifiers Annual » MC Publishing Corp., Ringwood, New Jersey, 1979 ; Dr. Helmut Stache : « Tensid Taschenbuch » Carl Hauser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

| 1. Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusöl-polyäthylenglykoläther (36 Mol AeO) | 5% | - | - |
| Tributylphenyl-polyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

**7. Stäubemittel**

| | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

**8. Extruder Granulat**

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

**9. Umhüllungs-Granulat**

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

**10. Suspensions-Konzentrat**

| | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1 : 1 R-cis-3-(2,2-Dichlorvinyl)-2,2-dimethylcyclopropankarbonsäure-R,S-α-methyl-(6-phenoxy-2-picolyl) methylester

Zu einer eisgekühlten Lösung von 3,2 g 1 R-cis-3-(2,2-Dichlorvinyl)-2,2-dimethylcyclopropankarbonsäurechlorid in 20 ml Toluol gibt man nacheinander 1,4 g Pyridin, gelöst in 5 ml Toluol und dann 3 g 1-(6-Phenoxy-2-pyridyl)-äthanol in 10 ml Toluol. Die leicht gelbliche Suspension versetzt man mit

200 mg 4-Dimethylaminopyridin und rührt das Reaktionsgemisch 16 Stunden bei Raumtemperatur. Nach Zugabe von 100 ml Toluol wird die organische Phase mit eisgekühlter 1N HCl-, 10 %iger $K_2CO_3$-, gesättigter $NaHCO_3$- und gesättigter Kochsalzlösung gewaschen und über Mg $SO_4$ getrocknet. Das Lösungsmittel wird nun unter vermindertem Druck entfernt und das Rohprodukt an Kieselgel mit Toluol als Eluiermittel gereinigt. Man erhält ein Diastereomeren (1 : 1) gemisch a) folgender Struktur

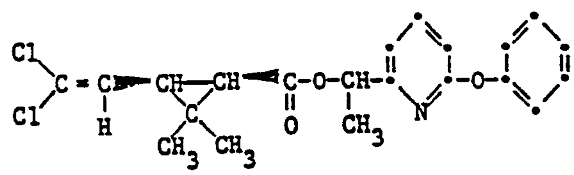

mit einem Brechungsindex von

$$n_D^{20°} = 1{,}544\,7$$

und einer optischen Rotation von

$$[\alpha]_D = + 23° \pm 1° \; [c = 1{,}06 \text{ in Benzol}]$$

Das Diastereomerengemisch wird chromatographisch in die optisch reinen Isomeren b) und c) aufgetrennt (Kieselgel, mit Toluol/Hexan = 95 : 5 als Eluiermittel).

Das Isomer b) wird dabei zuerst eluiert und besitzt folgende physikalische Daten

$$n_D^{20°} = 1{,}560\,5$$

$$[\alpha]_D = + 116° \pm 1° \; [c = 1{,}07 \text{ in Benzol}]$$

Das Isomer c) wird charakterisiert durch

$$n_D^{20°} = 1{,}559\,1$$

$$[\alpha]_D = — 69° \pm 1° \; [c = 0{,}937 \text{ in Benzol}]$$

Auf analoge Weise wird auch der 1 R-trans-3-(2,2-Dichlorvinyl)-2,2-dimethylcyclopropankarbonsäure-R,S-α-methyl-(6-phenoxy-2-picolyl)-ester d) mit einem Brechungsindex von

$$n_D^{20°} = 1{,}559\,1$$

und einer optischen Rotation von

$$[\alpha]_D = + 1° \; [c = 0, \text{ in Chloroform}]$$

hergestellt.

Das Diastereomerengemisch wird chromatographisch in die optisch reinen Isomeren e) und f) aufgetrennt.

Das Isomere e) hat folgende physikalische Daten

$$n_D^{21°} = 1{,}558\,1$$

$$[\alpha] = + 77° \; [c = 0{,}74 \text{ in Benzol}]$$

und das Isomere f)

$$n_D^{21°} = 1{,}558\,1$$

$$[\alpha] = — 56° \; [c = 0{,}83 \text{ in Benzol}]$$

Beispiel 2 : Insektizide Frassgift-Wirkung :

Baumwollpflanzen werden mit einer Versuchslösung, enthaltend 1,25 ; 0,6 oder 0,3 g der zu prüfenden Verbindung pro 100 1 $H_2O$, besprüht.

Nach dem Antrocknen des Belages werden die Pflanzen mit Larven der Spezies Heliothis virescens ($L_1$-Stadium) besetzt. Man verwendet pro Versuchsverbindung und zwei Pflanzen, und eine Auswertung

der erzielten Abtötungsrate erfolgt nach 24 und 48 Stunden. Der Versuch wird bei 24 °C und 60 % relativer Luftfeuchtigkeit durchgeführt.

Verbindungen gemäss dem Herstellungsbeispiel 1 zeigen die in der folgenden Tabelle angegebene Wirkung gegen Larven der Spezies Heliothis virescens :

Mortalität in % der Heliothis virescens $L_1$-Larven

| Konz. | 1,25 g | | 0,6 g | | 0,3 g | |
|---|---|---|---|---|---|---|
| Verbindungen | 24 | 48 | 24 | 48 | 24 | 48 Std. |
| a) | 50 | 83 | 38 | 60 | 20 | 33 |
| b) | 43 | 90 | 43 | 77 | 28 | 55 |
| Isomeren-gemisch der Formel g) * bekannt aus U.S.P. 4,323,574 | 15 | 20 | 0 | 0 | 0 | 0 |

* 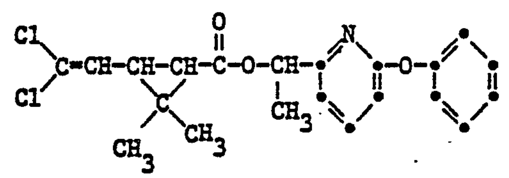 g) (cis/trans = 9:1)

Beispiel 3 : Wirkung gegen Zecken

A) Amblyomma hebraeum

50 Nymphen werden in ein Glasröhrchen gezählt und für 1 bis 2 Minuten in 2 ml einer wässrigen Emulsion aus einer Verdünnungsreihe mit je 10, 1 oder 0,1 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Auswertung erfolgt nach 1 Woche. Für jeden Versuch werden 2 Wiederholungen durchgeführt.

B) Boophilus microplus (Larven)

Mit einer analogen Verdünnungsreihe wie beim Test A) werden mit je 20 sensiblen resp. OPresistenten Larven Versuche durchgeführt. (Die Resistenz bezieht sich auf die Verträglichkeit von Diazinon).

Die Verbindungen gemäss dem Herstellungsbeispiel 1 zeigen die in der folgenden Tabelle angegebene Wirkung gegen Nymphen bzw. Larven der Zecken : Amblyomma hebraeum und Boophilus microplus.

Biologische Versuchsergebnisse

In der folgenden Tabelle sind Versuchsergebnisse auf der Basis des Beispiels 3 aufgeführt, und zwar mit folgendem Bewertungsindex in bezug auf die prozentuale Abtötung der Schädlinge :

A : 70-100 % Abtötung bei 0,1 ppm Wirkstoffkonzentration
B : 70-100 % Abtötung bei 1 ppm Wirkstoffkonzentration
C : 70-100 % Abtötung bei 10 ppm Wirkstoffkonzentration

| Verbindungen | Abtötung von<br>A.hebraeum Nymphen | <br>Boophilus sens. Larven | <br>OP resistente Larven |
|---|---|---|---|
| a) | A | A | A |
| b) | A | A | A |
| c) | B | B | B |
| d) | B | B | B |
| e) | B | B | C |
| f) | B | B | C |

**Patentansprüche** (für die Vertragsstaaten : BE, CH, DE, FR, GB, IT, LI, NL)

1. Diastereomere 1 R-cis-3-(2,2-Dichlorvinyl)-2,2-dimethylcyclopropankarbonsäure-R,S-α-methyl-(6-phenoxy-2-picolyl) ester der Formel I

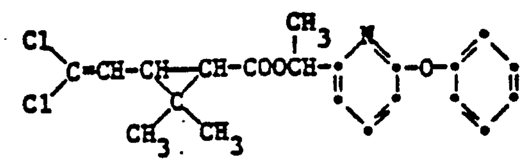

(I)

2. Der 1 R-cis-3-(2,2-Dichlorvinyl)-2,2-dimethylcyclopropankarbonsäure-R-α-methyl-(6-phenoxy-2-picolyl) ester gemäss Anspruch 1.

3. Der 1 R-cis-3-(2,2-Dichlorvinyl)-2,2-dimethylcyclopropankarbonsäure-S-α-methyl-(6-phenoxy-2-picolyl) ester gemäss Anspruch 1.

4. Diastereomere 1 R-trans-3-(2,2-Dichlorvinyl)-2,2-dimethylcyclopropankarbonsäure-R,S-α-methyl-(6-phenoxy-2-picolyl) ester der Formel I, Anspruch 1.

5. Der 1 R-trans-3-(2,2-Dichlorvinyl)-2,2-dimethylcyclopropankarbonsäure-R̂-α-methyl-(6-phenoxy-2-picolyl) ester gemäss Anspruch 4.

6. Der 1 R-trans-3-(2,2-Dichlorvinyl)-2,2-dimethylcyclopropankarbonsäure-S-α-methyl-(6-phenoxy-2-picolyl) ester gemäss Anspruch 4.

7. Ein Verfahren zur Herstellung von Verbindungen gemäss den Ansprüchen 1-6, dadurch gekennzeichnet, dass man in Gegenwart eines säurebindenden oder wasserbindenden Mittels eine Verbindung der Formel

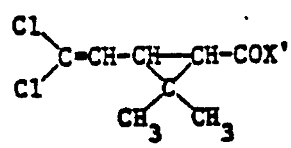

mit einer Verbindung der Formel

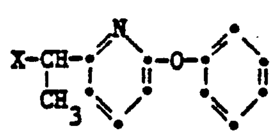

umsetzt, worin die Symbole X und X' je für eine Hydroxylgruppe oder eines für eine Hydroxylgruppe und das andere für ein Halogenatom steht, und anschliessend die entstandenen Diastereomerengemische chromatographisch auftrennt.

8. Ein Schädlingsbekämpfungsmittel, dadurch gekennzeichnet, dass es neben geeigneten Träger- und/oder anderen Zuschlagstoffen als aktive Komponente eine Verbindung gemäss Anspruch 1 oder Anspruch 4 enthält.

**Patentansprüche** (für der Vertragsstaat AT)

1. Ein Schädlingsbekämpfungsmittel, dadurch gekennzeichnet, dass es neben geeigneten Träger-und/oder anderen Zuschlagstoffen als aktive Komponente die diastereomeren 1 R-cis-3-(2,2-Dichlorvinyl)-2,2-dimethylcyclopropankarbonsäure-R,S-α-methyl-(6-phenoxy-2-picolyl) ester der Formel

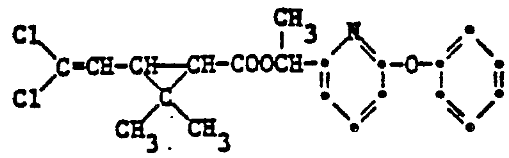

enthält.

2. Mittel gemäss Anspruch 1, dadurch gekennzeichnet, dass es als aktive Komponente den 1 R-cis-3-(2,2-Dichlorvinyl)-2,2-dimethylcyclopropankarbonsäure-R-α-methyl-(6-phenoxy-2-picolyl) ester enthält.

3. Mittel gemäss Anspruch 1, dadurch gekennzeichnet, dass es als aktive Komponente den 1 R-cis-3-(2,2-Dichlorvinyl)-2,2-dimethylcyclopropankarbonsäure-S-α-methyl-(6-phenoxy-2-picolyl) ester enthält.

4. Ein Schädlingsbekämpfungsmittel, dadurch gekennzeichnet, dass es neben geeigneten Träger- und/oder anderen Zuschlagstoffen als aktive Komponente die diastereomeren 1 R-trans-3-(2,2-Dichlorvinyl)-2,2-dimethylcyclopropankarbonsäure-R,S-α-methyl-(6-phenoxy-2-picolyl) ester enthält.

5. Mittel gemäss Anspruch 4, dadurch gekennzeichnet, dass es als aktive Komponente den 1 R-trans-3-(2,2-Dichlorvinyl)-2,2-dimethylcyclopropankarbonsäure-R-α-methyl-(6-phenoxy-2-picolyl) ester enthält.

6. Mittel gemäss Anspruch 4, dadurch gekennzeichnet, dass es als aktive Komponente den 1 R-trans-3-(2,2-Dichlorvinyl)-2,2-dimethylcyclopropankarbonsäure-S-α-methyl-(6-phenoxy-2-picolyl) ester enthält.

7. Ein Verfahren zur Herstellung von Verbindungen gemäss den Ansprüchen 1-6, dadurch gekennzeichnet, dass man in Gegenwart eines säurebindenden oder wasserbindenden Mittels eine Verbindung der Formel

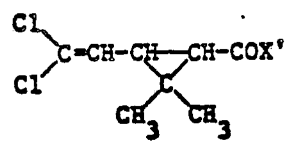

mit einer Verbindung der Formel

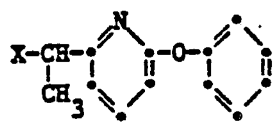

umsetzt, worin die Symbole X und X' je für eine Hydroxylgruppe oder eines für eine Hydroxylgruppe und das andere für ein Halogenatom steht, und anschliessend die entstandenen Diastereomerengemische chromatographisch auftrennt.

**Claims** (for the Contracting States : BE, CH, DE, FR, GB, IT, LI, NL)

1. Diastereoisomeric 1 R-cis-3-(2,2-Dichlorovinyl)-2,2-dimethylcyclopropanecarboxylic acid-R,S-α-methyl-(6-phenoxy-2-picolyl) esters of the formula I

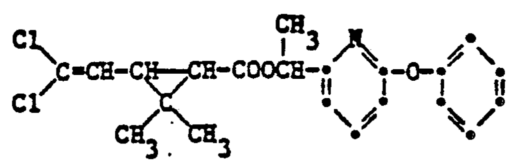

(I)

2. 1 R-cis-3-(2,2-Dichlorovinyl)-2,2-dimethylcyclopropanecarboxylic acid-R-α-methyl-(6-phenoxy-2-picolyl) ester according to Claim 1.

3. 1 R-cis-3-(2,2-Dichlorovinyl)-2,2-dimethylcyclopropanecarboxylic acid-S-α-methyl-(6-phenoxy-2-picolyl) ester according to Claim 1.

4. Diastereoisomeric 1 R-trans-3-(2,2-dichlorovinyl)-2,2-dimethylcyclopropanecarboxylic acid-R,S-α-methyl-(6-phenoxy-2-picolyl) esters of the formula I, according to Claim 1.

5. 1 R-trans-3-(2,2-Dichlorovinyl)-2,2-dimethylcyclopropanecarboxylic acid-R-α-methyl-(6-phenoxy-2-picolyl) ester according to Claim 4.

6. 1 R-trans-3-(2,2-Dichlorovinyl)-2,2-dimethylcyclopropanecarboxylic acid-S-α-methyl-(6-phenoxy-2-picolyl) ester according to Claim 4.

7. A process for producing compounds according to Claims 1 to 6, characterised in that a compound

of the formula

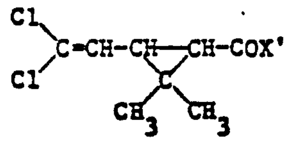

is reacted in the presence of an acid-binding agent or water-binding agent with a compound of the formula

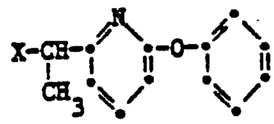

wherein the symbols X and X′ are each a hydroxyl group, or one is a hydroxyl group and the other a halogen atom, and subsequently the diastereoisomeric mixtures formed are separated by chromatography.

8. A pesticidal composition, characterised in that it contains as active ingredient a compound according to Claim 1 or Claim 4 together with suitable carriers and/or other additives.

**Claims** (for the Contracting State AT)

1. A pesticidal composition, characterised in that it contains as active ingredient the diastereoisomeric 1 R-cis-3-(2,2-dichlorovinyl)-2,2-dimethylcyclopropanecarboxylic acid-R,S-α-methyl-(6-phenoxy-2-picolyl) esters of the formula

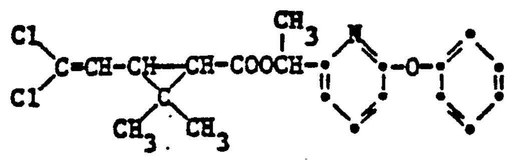

(I)

together with suitable carriers and/or other additives.

2. A composition according to Claim 1, characterised in that it contains as active ingredient 1 R-cis-3-(2,2-dichlorovinyl)-2,2-dimethylcyclopropanecarboxylic acid-R-α-methyl-(6-phenoxy-2-picolyl) ester.

3. A composition according to Claim 1, characterised in that it contains as active ingredient 1 R-cis-3-(2,2-dichlorovinyl)-2,2-dimethylcyclopropanecarboxylic acid-S-α-methyl-(6-phenoxy-2-picolyl) ester.

4. A pesticidal composition, characterised in that it contains as active ingredient diastereoisomeric 1 R-trans-3-(2,2-dichlorovinyl)-2,2-dimethylcyclopropanecarboxylic acid-R,S-α-methyl-(6-phenoxy-2-picolyl) esters together with suitable carriers and/or other additives.

5. A composition according to Claim 4, characterised in that it contains as active ingredient 1 R-trans-3-(2,2-dichlorovinyl)-2,2-dimethylcyclopropanecarboxylic acid-R-α-methyl-(6-phenoxy-2-picolyl) ester.

6. A composition according to Claim 4, characterised in that it contains as active ingredient 1 R-trans-3-(2,2-dichlorovinyl)-2,2-dimethylcyclopropanecarboxylic acid-S-α-methyl-(6-phenoxy-2-picolyl) ester.

7. A process for producing compounds according to Claims 1 to 6, characterised in that a compound of the formula

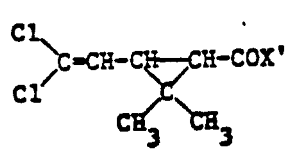

is reacted in the presence of an acid-binding agent or water-binding agent with a compound of the formula

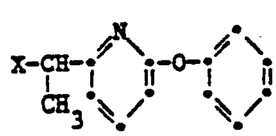

wherein the symbols X and X' are each a hydroxyl group, or one is a hydroxyl group and the other a halogen atom, and subsequently the diastereoisomeric mixtures formed are separated by chromatography.

**Revendications** (pour les Etats Contractants : BE, CH, DE, FR, GB, IT, LI, NL)

1. 1 R-cis-3-(2,2-dichlorovinyl)-2,2-diméthylcyclopropanecarboxylate de R,S-α-méthyl-(6-phénoxy-2-picolyle) diastéréomères répondant à la formule I

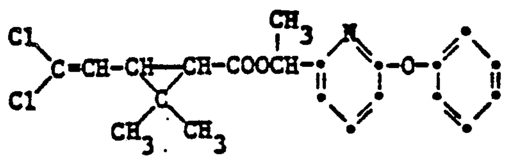

(I)

2. 1 R-cis-3-(2,2-dichlorovinyl)-2,2-diméthylcyclopropanecarboxylate de R-α-méthyl-(6-phénoxy-2-picolyle) selon la revendication 1.
3. 1 R-cis-3-(2,2-dichlorovinyl)-2,2-diméthylcyclopropanecarboxylate de S-α-méthyl-(6-phénoxy-2-picolyle) selon la revendication 1.
4. 1 R-trans-3-(2,2-dichlorovinyl)-2,2-diméthylcyclopropanecarboxylate de R,S-α-méthyl-(6-phénoxy-2-picolyle) diastéréomères répondant à la formule I, revendication 1.
5. 1 R-trans-3-(2,2-dichlorovinyl)-2,2-diméthylcyclopropanecarboxylate de R-α-méthyl-(6-phénoxy-2-picolyle) selon la revendication 4.
6. 1 R-trans-3-(2,2-dichlorovinyl)-2,2-diméthylcyclopropanecarboxylate de S-α-méthyl-(6-phénoxy-2-picolyle) selon la revendication 4.
7. Procédé de préparation de composés selon les revendications 1 à 6, caractérisé en ce qu'on fait réagir en présence d'un agent de fixation des acides ou de fixation de l'eau un composé répondant à la formule

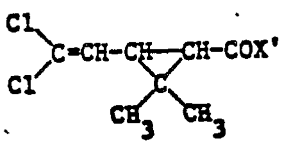

avec un composé répondant à la formule

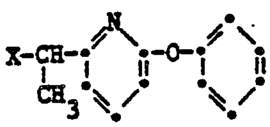

dans laquelle les symboles X et X' représentent chacun un groupe hydroxyle ou l'un un groupe hydroxyle et l'autre un atome d'halogène, puis on sépare chromatographiquement les mélanges de diastéréomères formés.
8. Agent de lutte contre les parasites, caractérisé en ce qu'il contient comme constituant actif, en plus de substances de support et/ou d'autres additifs appropriés, un composé selon la revendication 1 ou la revendication 4.

**Revendications** (pour l'Etat contractant AT)

1. Agent de lutte contre les parasites, caractérisé en ce qu'il contient comme constituant actif, en plus de substances de support et/ou d'autres additifs appropriés, les 1 R-cis-3-(2,2-dichlorovinyl)-2,2-diméthylcyclopropanecarboxylate de R,S-α-méthyl-(6-phénoxy-2-picolyle) diastéréomères répondant à la formule

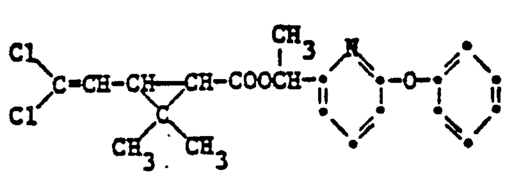

(I)

2. Agent selon la revendication 1, caractérisé en ce qu'il contient comme constituant actif le 1 R-cis-3-(2,2-dichlorovinyl)-2,2-diméthylcyclopropanecarboxylate de R-α-méthyl-(6-phénoxy-2-picolyle).

3. Agent selon la revendication 1, caractérisé en ce qu'il contient comme constituant actif le 1 R-cis-3-(2,2-dichlorovinyl)-2,2-diméthylcyclopropanecarboxylate de S-α-méthyl-(6-phénoxy-2-picolyle).

4. Agent de lutte contre les parasites, caractérisé en ce qu'il contient comme constituant actif, en plus de substances de support et/ou d'autres additifs appropriés, les 1 R-trans-3-(2,2-dichlorovinyl)-2,2-diméthylcyclopropanecarboxylate de R,S-α-méthyl-(6-phénoxy-2-picolyle) diastéréomères.

5. Agent selon la revendication 4, caractérisé en ce qu'il contient comme constituant actif le 1 R-trans-3-(2,2-dichlorovinyl)-2,2-diméthylcyclopropanecarboxylate de R-α-méthyl-(6-phénoxy-2-picolyle).

6. Agent selon la revendication 4, caractérisé en ce qu'il contient comme constituant actif le 1 R-trans-3-(2,2-dichlorovinyl)-2,2-diméthylcyclopropanecarboxylate de S-α-méthyl-(6-phénoxy-2-picolyle).

7. Procédé de préparation de composés selon les revendications 1 à 6, caractérisé en ce qu'on fait réagir en présence d'un agent de fixation des acides ou de fixation de l'eau un composé répondant à la formule

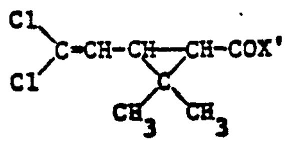

avec un composé répondant à la formule

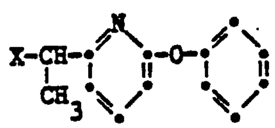

dans laquelle les symboles X et X' représentent chacun un groupe hydroxyle ou l'un un groupe hydroxyle et l'autre un atome d'halogène, puis on sépare chromatographiquement les mélanges de diastéréomères formés.